# EUROPÄISCHE PATENTSCHRIFT

(11) **EP 0 652 762 B1**
(45) Veröffentlichungstag und Bekanntmachung des Hinweises auf die Patenterteilung: **30.12.1998**
(21) Anmeldenummer: 94916986.6
(22) Anmeldetag: 24.05.1994
(51) Int. Cl.: A61K 35/78

(54) **NEUARTIGES ANXIOLYTIKUM**
NOVEL ANXIOLYTIC
ANXIOLYTIQUE D'UN TYPE NOUVEAU

(30) Priorität: 28.05.1993 DE 4317868
(43) Veröffentlichungstag der Anmeldung: 17.05.1995
(73) Patentinhaber: ARROWDEAN LTD., IE-Dublin 2 (IE)
(72) Erfinder: HÄCKER, Rüdiger, D-82211 Herrsching (DE); MATTERN, Claudia, D-82335 Berg (DE)
(74) Vertreter: Winkler, Andreas, Dr.
(86) Internationale Anmeldenummer: EP9401690
(87) Internationale Veröffentlichungsnummer: WO9427625

(56) Entgegenhaltungen:
- DE-A- 3 626 128

## Beschreibung

Die Erfindung betrifft ein neuartiges Anxiolytikum.

Angst und Angstzustände sind typisch menschliche Erscheinungen, die durch ihre Folgeerscheinungen in Form von Verhaltensänderungen und Störungen im vegetativen Nervensystem zu einer erheblichen Beeinträchtigung der Lebensqualität führen. Ausgelöst durch Überbelastungen, Zwangssituationen, Fehlleistungen im beruflichen und privaten Bereich und andere Streßbelastungen werden typische psychosomatische Symptome manifest. Dazu zählen Flucht- und Vermeidungsreaktionen, aber auch Störungen im vegetativen Bereich, wie Magen- und Darmbeschwerden oder der sprichwörtliche "Kloß im Hals".

Bei Kumulation der auslösenden Faktoren kann ein psychovegetatives Erschöpfungssyndrom entstehen, das durch Leistungsabfall mit psychischen und vegetativen Störungen wie Kopfschmerzen, Magenbeschwerden, Konzentrationsschwäche, Reizbarkeit und Schlafstörungen gekennzeichnet ist.

Die biochemischen, pharmakologischen und neurophysiologischen Mechanismen, die diesem Syndrom zugrundeliegen, sind nicht vollständig aufgeklärt und wahrscheinlich auch nicht einheitlich. Grundsätzlich kann davon ausgegangen werden, daß die Homöostase der Neurotransmittersysteme Noradrenalin, Dopamin und 5-Hydroxytryptamin gestört ist. Weiterhin wird vermutet, daß neben der Wirkung über die monoaminergen Synapsen, die endogene γ-Aminobuttersäure bzw. mit ihr in Wechselwirkung stehende Transmittersysteme an der Auslösung der Symptome beteiligt sind. Es liegen Hinweise vor, daß in solchen Situationen die Blut-Hirn-Schranke für niedermolekulare Peptide durchlässiger wird. Damit können durch solche Peptide Wirkungen auf zentralnervaler Ebene ausgelöst werden.

Zur Therapie der psychoreaktiven Störungen werden heute ausschließlich Arzneimittel eingesetzt, die der Gruppe der Psychopharmaka zuzurechnen sind. Die bevorzugte Gruppe sind die Tranquilizer, auch als Anxiolytika bezeichnet, mit den weltweit am häufigsten in diesem Bereich verwendeten Benzodiazepinen und ihren Derivaten. Diese Wirkstoffe greifen insofern in den Stoffwechsel der endogenen γ-Aminobuttersäure ein, als sie mit spezifischen Bindungsstellen reagieren, die zu einem Komplex gehören, der aus GABA-Rezeptor, Benzodiazepin-Rezeptor und einen Ionenkanal für Chloridionen besteht. Diese Rezeptoren sind im zentralen Nervensystem überall vorhanden.

Je nach Ausprägung der Symptome finden auch Neuroleptika Anwendung, die regularisierend in den Monoaminstoffwechsel eingreifen, wie die Phenothiazine. Bei Mangel an Catecholaminen und Serotonin (5-Hydroxytryptamin) können mit Derivaten des Phenothiazins, den trizyklischen Antidepressiva, therapeutische Erfolge erzielt werden.

Im Bereich der Phytopharmaka werden nur dem Johanniskraut antidepressive Wirkungen zugeschrieben. Im Vordergrund steht eine aufhellende Wirkung auf die Stimmungslage, die Aussagen zu neurovegetativen Wirkungen sind nicht einheitlich und zum Teil strittig.

Als Nachteil der bisherigen medikamentösen Behandlung ist generell anzusehen, daß die Tranquilizer, aber auch die Neuoleptika und Antidepressiva neben der gewünschten Hauptwirkung, der Anxiolyse, eine Reihe von unerwünschten Nebenwirkungen entfalten. Dazu zählt insbesondere die Sedierung, die zu Müdigkeit und erhöhtem schlafbedürfnis führt. Die muskelrelaxierende Wirkung kann sich bei hohen Dosierungen ungünstig auswirken. Durch die in der Regel länger dauernde Therapie tritt eine erhebliche Belastung des Leberstoffwechsels ein.

Weiterhin stehen Tranquilizer in enger Wechselwirkung mit anderen zentralwirksamen Pharmaka, wie Hypnotika, aber auch Analgetika und Stimulantien sowie Alkohol. In der Regel werden die Wirkungen dieser Substanzen verstärkt, es treten z.T. sogar Effekte auf, die nach Verabreichung der Einzelsubstanzen nicht oder nur in sehr geringem Ausmaß zu bemerken sind.

Diese Situation hat dazu geführt, daß weltweit nach weiteren Substanzen bzw. Derivaten bekannter Wirkstoffe gesucht wird, um diese Nachteile zu minimieren. Der vorliegenden Erfindung liegt die Aufgabe zugrunde, diesem Bedürfnis nachzukommen.

Erfindungsgemäß wird diese Augabe durch die Verwendung einer Zusammensetzung mit einem Gehalt an Wirkstoffen aus Rhizoma zingiberis und Ginkgo bilobae zur Herstellung eines Arzneimittels zur Behandlung von Angstzuständen gelöst.

Dabei ist bevorzugt, daß die Zusammensetzung eine Kombination aus Extrakten aus Rhizoma zingiberis und Ginkgo bilobae enthält.

Die Erfindung schlägt vor daß die Wirkstoffe bzw. Extrakte in einer Einnahmeeinheit in Kapselform oder anderer festen oder flüssigen Arzneiform, die für die perorale Applikation geeignet ist vorliegen.

Bei der erfindungsgemäßen Verwendung liegt der bevorzugte Gehalt an Extrakt aus Rhizoma zingiberis zwischen 50 und 200 mg, noch bevorzugter bei 100 mg pro Einnahmeeinheit. Der Gehalt an Extrakt aus Ginkgo bilobae liegt bevorzugt zwischen 10 und 100 mg, noch bevorzugter bei 40 mg pro Einnahmeeinheit. Die Erfindung sieht in einer bevorzugten Ausführungsform vor, daß der Extrakt aus Rhizoma zingiberis durch eine Ethanol/Wasser- oder Aceton/Wasser-Extraktion gewonnen ist und im wesentlichen als Oleoresin vorliegt.

Die Erfindung sieht alternativ vor, daß der Extrakt aus Rhizoma zingiberis durch Extraktion mit CO₂ gewonnen ist.

Dabei kann das Oleoresin oder der CO₂-Extrakt aus Rhizoma zingiberis durch Mikroverkapselung, Granulierung oder Lyophilisierung in eine geeignete galenische Form überführt sein.

Das erfindungsgemäße Kombinationspräparat ist als solches bereits aus der DE 36 26 128 C2 als Arzneimittel gegen Übelkeit und Erbrechen bekannt geworden. Völlig überraschend hat sich bei neueren klinischen Untersuchungen herausgestellt, daß diese Wirkstoffkombination bei geeigneter Zusammensetzung auch eine anxiolytische Wirkung entfaltet, die weitgehend von den geschilderten Nebenwirkungen, insbesondere der Benzodiazepine, frei ist.

Diese Wirkung ist insofern auch für den Fachmann überraschend, da sie sich aus den bisher bekannten Wirkungen sowohl der beiden Einzelextrakte als auch des Kombinationspräparates und den zugrundeliegenden biochemischen Mechanismen nicht herleiten läßt.

Der Ingwerwurzelstock und aus Ingwer hergestellte Arzneimittel (Pulver, Tinkturen, Extrakte, Aufgüsse) besitzen eine starke antiemetische Wirkung, unterdrücken den Hustenreflex und sind als Stomachikum, Tonikum und Digestivum bei Appetitlosigkeit, subazider Gastritis und Dyspepsien bekannt.

Außerdem werden Ingwer eine kardiotone (positiv inotrope) Wirkung sowie eine Begünstigung der Zirkulation und positive Effekte auf die Absorption und Verteilung anderer Arzneimittel zugeschrieben. Der Verdauungsprozeß wird durch Ingwer auch durch die Beschleunigung des Gallenfluxes und den natürlichen Gehalt an Proteasen gefördert. Eine Hemmung der Prostaglandin-Biosynthese soll ebenfalls ausgelöst werden.

Insgesamt ergibt sich aus der Literatur in etwa folgendes Wirkungsspektrum:

Ein ethanolischer Extrakt von Rhizoma zingiberis weist folgende pharmakodynamische Eigenschaften auf:
- antiserotoninerge Wirkungen mit besonderer Affinität zum 5-HT-3-Rezeptor auf peripherer Ebene
- Hemmung der Freisetzung von Prostaglandinen aus Leukozyten
- Hemmung der Thrombozytenaggregation
- Regularisierung der Prostaglandinsynthese
- Beeinflussung vasomotorischer Zentren, Normalisierung der zentralnervalen Durchblutung.

Aus diesem Wirkungsspektrum heraus läßt sich ein anxiolytischer Effekt nicht erwarten.

Der Ginkgo-Extrakt verfügt über ein differenziertes pharmakologisches Wirkprofil, das metabolische und hormonelle Regulationsmechanismen einbezieht, die die Eigenschaften von Membranen, Gefäßwänden, Blut und Gewebeflüssigkeiten beeinflussen.

Als Indikationsgebiete haben sich durchgesetzt:
- periphere und arterielle Durchblutungsstörungen bei noch erhaltener Durchblutungsreserve
- Hirnleistungsstörungen mit nachlassender intellektueller Leistungsfähigkeit, besonders beim alternden Patienten
- Störungen im Bereich des Labyrinthsystems.

Hinweise auf anxiolytische Wirkungen finden sich in der umfangreichen Literatur nicht.

Offensichtlich ergibt die Kombination beider Extrakte - wie erfindungsgemäß vorgeschlagen - eine neuartige koergistische Wirkung, die besonders geeignet ist, psychovegetative Störungen, die auf Angstzuständen und Streßsituationen beruhen, wie z.B. das psychovegetative Erschöpfungssyndrom, zu therapieren, und vermutlich durch die Beeinflussung peripherer und zentraler Regelkreise zustande kommt.

Eine bevorzugte Ausführungsform der Kombination ergibt sich aus der Verwendung von Extrakten aus Rhizoma zingiberis und Ginkgo bilobae, die durch kombinierte Extraktion mit Ethanol/Wasser oder Azeton/Wasser-Gemischen oder CO₂ gewonnen werden können. Diese Extrakte sind auf die wesentlichen bekannten Inhaltsstoffe standardisiert.

Besonders bevorzugt ist die Mischung von 100 mg Extrakt (Oleoresin oder CO₂-Extrakt) aus Rhizoma zingiberis und 40 mg Standard-Extrakt aus Ginkgo bilobae, die als Kapsel appliziert wird. Als tägliche Gesamtdosis werden entsprechend dem nachfolgenden Analysebeispiel 3-4 Einnahmeeinheiten (300-400 mg Extrakt aus Rhizoma zingiberis und 120-160 mg Extrakt aus Ginkgo bilobae) empfohlen.

Die erfindungsgemäße neue Indikation des Kombinationspräparates zeichnet sich dadurch aus, daß vegetative Störungen im Bereich der glatten Muskulatur (Magen-Darm-Kanal, Gefühl der Enge im Hals, etc.) ebenso beseitigt werden, wie die begleitenden Kopfschmerzen, Reizbarkeit und Schlafstörungen. Besonders hervorzuheben ist, daß weder Müdigkeit noch das Gefühl der Schlaffheit der Muskulatur auftreten, sondern im Gegenteil über Verbesserung der Spannkraft und Vigilanz berichtet wird.

Ein weiterer Vorteil der neuen Indikation des bekannten Kombinationspräparates ist seine pflanzliche Herkunft. Daraus resultiert eine extrem niedrige, praktisch nicht vorhandene Toxizität und das völlige Fehlen kanzerogener oder mutagener Wirkungen.

### Kurze Beschreibung der Zeichnung

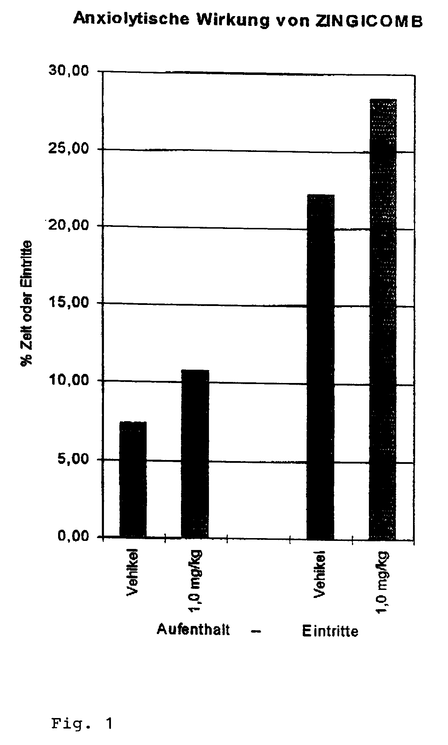

- Fig. 1: zeigt die anxiolytische Wirkung bei Applikation des erfindungsgemäßen Präparates gemäß folgendem Herstellungsbeispiel im Vergleich mit der Applikation des Vehikels allein.

### Herstellungsbeispiel

Extrakt aus Rhizoma zingiberis wird durch Ethanol/Wasser-Extraktion und anschließendes Einengen bis zu einem Oleoresin oder durch CO₂-Extraktion gewonnen. Nach üblichen standardisierten Verfahren wird ein ähnlicher Extrakt aus Ginkgo bilobae gewonnen.

Die pulverförmigen Extrakte werden im Gewichtsverhältnis (Rhizoma zingiberis: Ginkgo bilobae) 10:4 gründlich miteinander vermischt und mit den bekannten galenischen Verfahren zu Kapseln verarbeitet, die jeweils ca. 100 mg Extrakt aus Rhizoma zingiberis und ca. 40 mg Extrakt aus Ginkgo bilobae enthalten.

Wenn es die galenische Formulierung erfordert, wird das Oleoresin oder der CO₂-Extrakt durch Mikroverkapselung oder Lyophilisierung in Pulverform überführt.

### Anwendungsbeispiel

Die Vorteile der Erfindung werden an folgendem Fallbeispiel sowie tierexperimentellen Untersuchungen demonstriert:

### Kasuistik:

Ein leitender Mitarbeiter einer Forschungs- und Entwicklungsabteilung, 51 Jahre alt, 168 m , 69 kg, sucht den Hausarzt mit den typischen Zeichen eines psychovegetativen Erschöpfungssyndroms auf.

### Anamese:

Der Patient ist zur Zeit für drei unterschiedliche umfangreiche Projekte verantwortlich. Zwei Projekte sind mit erheblichem Arbeits- und Organisationsaufwand verbunden, die eine Vielzahl von Entscheidungen mit kurz- bis mittelfristigen Aktivitäten verlangen. Diese Projekte sind stark termingebunden und auch finanziell noch nicht klar abgesichert. Das dritte Projekt steht zwar nicht unter Termindruck, erfordert aber strategische Planungen, die weit in die Zukunft orientiert sind.

Der Patient klagt seit 14 Tagen über unruhigen Schlaf. Er wacht häufig auf, verspürt einen Druck im Magen, denkt über die Projekte nach. Tagsüber ißt er unregelmäßig, ist stark auf die Aufgabe konzentriert, konsumiert große Mengen Kaffee. Wenn er am Abend eine größere Mahlzeit zu sich nimmt, verspürt er wenig Appetit, hat Magenbeschwerden nach Nahrungsaufnahme.

Als in einer entscheidenden Phase der beiden Projekte Probleme im Personalbereich hinzukommen, verschlechtert sich die Situation. Der Patient hat das Gefühl, sich nicht mehr ausreichend auf wesentliche Dinge konzentrieren zu können, zweifelt an seiner Kreativität. Morgens verspürt er bei Überdenken der Tagesaufgaben und möglicher Konfliktsituationen Übelkeit, verbunden mit Kopfschmerzen und Schwindelgefühlen.

Er sucht zögernd den Arzt auf, da ihm die Nebenwirkungen von Psychopharmaka bekannt sind und er befürchtet, daß sie seine gegenwärtige Situation verschlechtern und nicht verbessern. Da der Patient beruflich Zugang zu phytopharmazeutischen Extrakten hat, schlägt er ihre Anwendung vor.

### Therapie:

Der Hausarzt verordnet eine zweitägige Ruhepause und billigt die dreimalige tägliche Applikation der besonders bevorzugten Ausführungsform des erfindungsgemäß eingesetzten Arzneimittels, da die Arzneistoffe an sich bekannt und monographiert sind.

Schon am nächsten Tag berichtet der Patient über das Ausbleiben des Völlegefühls, einen ungestörten Schlaf und das Fehlen der morgendlichen Übelkeit.

Er verträgt seine Mahlzeiten gut, reduziert den Kaffeekonsum und hat den Eindruck, daß ihm die Konzentration auf das strategische Projekt leichter fällt. Es treten keine Kopfschmerzen mehr auf.

Am nächsten Tag verstärkt sich dieser Eindruck, er berichtet über steigende Zuversicht und hat das Gefühl, wieder leistungsfähiger zu sein. Ein Überdenken seiner Situation führt ihn zu Schlußfolgerungen, die ihn zu seiner offensiven Haltung gegenüber

Problemen und seiner Entschluß- und Risikofreudigkeit zurückführen. Der Hausarzt empfiehlt darauf die Medikation auf einmal täglich zu reduzieren und sie nach zwei weiteren Tagen vollständig abzusetzen.

### Tierexperimentelle Untersuchungen

Die Studie wurde mit einem Verhaltensmodell durchgeführt, das für die Tiere eine Konfliktsituation zwischen dem Drang nach Erkundung der Umwelt und der angeborenen Furcht vor den Gefahren einer offenen Umwelt stimuliert.

Das Modell wurde 1984 von Handley und Mithani entwickelt (Effects of a2-adrenoceptor agonists and antagonists in a maze exploration model of "fear" - motivated behaviour, Naunyn-Schmiedeberg's Arch. Pharmacol. 327, 1-5 (1984)). Es besteht aus einem Kreuz mit gleich langen Armen (in Form eines + Zeichens). Zwei dieser Arme sind mit Wänden versehen, zwei Arme sind offen, ohne Wände (Fachausdruck in der englischen Literatur "+ maze" oder "plus-maze"). Die Modellanordnung befindet sich frei in einer bestimmten Höhe über dem Fußboden.

Die Bewertung des Verhaltens geht von der Beobachtung von Handley und Mithani aus, daß Ratten die offenen Arme vermeiden und die geschlossenen Arme für einen Aufenthalt bevorzugen. Das Ausmaß der Furcht (anxiety) kann daher sowohl durch die Zahl der Eintritte in die offenen Arme im Verhältnis zu den gesamten Eintritten in die Arme ausgedrückt werden (offen: total ratio, OTR), als auch durch den Prozentsatz an Zeit, für den sich die Tiere in den offenen Armen aufhalten.

Das Modell zeigt sowohl für klassische Anxiolytika wie Benzodiazeptine, Barbiturate und Ethanol als auch für anxiogene Stoffe wie Yohimbin und Pentylentetrazol typische und validerte Effekte, die in der Literatur beschrieben sind (Übersicht bei Handley et al. aaO).

In der Studie wurde das erfindungsgemäße Präparat (siehe Herstellungsbeispiel), kurz ZINGICOMB, intragastral appliziert und die Ergebnisse mit den Effekten verglichen, die bei alleiniger Applikation des Vehikels erhalten wurden.

### Versuchstiere

Als Versuchstiere wurden männliche Wistar-Ratten mit einem Gewicht von 250-300 g verwendet. Die Ratten wurden in Gruppen von je 5 in separaten Käfigschalen gehalten und hatten freien Zugang zu Futter und Wasser. Der Raum hatte einen Licht/Dunkel-Zyklus von 12 zu 12 Stunden.

### Verhaltensmodell

Das Modell entsprach dem Typ des "elevated plus-maze" der Literatur und hatte zwei offene Arme mit den Maßen 50 x 10 cm und zwei geschlossene Arme (Maße 50 x 10 x 40 cm). Die beiden Arme eines jeden Typs lagen sich gegenüber. Das gesamte Modell befand sich frei 50 cm über dem Fußboden.

Das Verhalten der Tiere wurde mit einem Videosystem aufgezeichnet, die Bänder wurden mit einem Computersystem hinsichtlich Anzahl der Eintritte in die Arme und Aufenthaltszeit in den Armen ausgewertet.

Die gesamte Anordnung befand sich in einem 2 x 2 m großen Raum, der mit einer Leistung von 15 W beleuchtet wurde.

### Applikation des Präparates

ZINGICOMB wurde durch Homogenisierung mit Ultraschall am Testtag in Wasser so suspendiert, daß bei einer Zufuhr von 2 ml/kg eine Dosierung von 1,0 mg/kg erhalten wurde. Die Applikation erfolgte intragastral mittels einer Sonde.

### Ablauf der Tests

Am Testtag wurden die Ratten in individuelle Käfigschalen umgesetzt, gewogen und das Vehikel oder das Präparat mittels einer Magensonde appliziert. Nach 60 min wurden die Tiere individuell in den Untersuchungsraum verbracht und in das Zentrum des Verhaltensmodells gesetzt, wobei das Gesicht auf einen der geschlossenen Arme gerichtet war. Während der folgenden 5 Minuten wurden über das Videosystem mit Computer außerhalb des Raumes die Zahl der Eintritte und die Zeit des Aufenthaltes in den geschlossenen und offenen Armen registriert.

### Ergebnisse

Die Abbildung zeigt die Resultate bezüglich der Aufenthaltszeit in den offenen Armen und die Anzahl der Eintritte in die offenen Arme als Prozentsatz aller Eintritte in Arme (Mittelwert). Angegeben ist der Mittelwert des Prozentsatzes der Gesamtzeit (5 min), zu dem sich die Tiere in den offenen Armen aufhielten. Ein anxiolytischer Effekt sollte auf der Grundlage der Theorie und Validierung des Modells zu einer Zunahme des Prozentsatzes führen.

Die Resultate weisen im Sinne dieser Erwartung einen anxiolytischen Effekt von ZINGICOMB nach. Die Dosis von 1,0 mg/kg führt zu einem deutlichen Anstieg der Verweilzeit um etwa 46 % im Vergleich zu den Zeiten, die bei alleiniger Applikation des Vehikels erreicht werden (Fig. 1).

Die Daten zur Anzahl der Eintritte in die Arme belegen ebenfalls, daß die Dosis von 1,0 mg/kg ZINGICOMB die Attraktivität der offenen Arme im Vergleich zum Vehikel deutlich erhöht. Die Eintritte sind ca. 28 % häufiger, als ohne Applikation von ZINGICOMB.

Die Zusammenfassung der erhobenen Befunde ergibt trotz der geringen Anzahl der getesten Tiere pro Dosis (1,0 mg, n = 6, Vehikel n = 12) eine signifikante Wirkung auf das Verhalten im Modell bei Applikationen von ZINGICOMB im Vergleich zur alleinigen Applikation des Vehikels.

Durch den erfindungsgemäßen neuartigen Einsatz des geschilderten Kombinationspräparates ist eine schnelle und problemlose Therapierung von Angst und Angstzuständen, insbesondere bei psychovegetativem Erschöpfungssyndrom, zu erwarten, wobei die

Nachteile der bekannten Tranquilizer, wie Sedierung, Erschlaffung und Müdigkeit, die den Therapieerfolg in vielen Fällen gefährden würden, nicht zu erwarten sind wie in den bisherigen Untersuchungen nicht auftraten.

Die in der vorstehenden Beschreibung, in den Ansprüchen und in der Zeichnung offenbarten Merkmale der Erfindung können sowohl einzeln als auch in beliebiger Kombination für die Verwirklichung der Erfindung in ihren verschiedenen Ausführungsformen wesentlich sein.

## Patentansprüche

1. Verwendung einer Zusammensetzung mit einem Gehalt an Wirkstoffen aus Rhizoma zingiberis und Ginkgo bilobae zur Herstellung eines Arzneimittels zur Behandlung von Angstzuständen.

2. Verwendung nach Anspruch 1, dadurch gekennzeichnet, daß die Zusammensetzung eine Kombination aus Extrakten aus Rhizoma zingiberis und Ginkgo bilobae enthält.

3. Verwendung nach Anspruch 2, dadurch gekennzeichnet, daß die Wirkstoffe bzw. Extrakte in einer Einnahmeeinheit in Kapselform oder anderer festen oder flüssigen Arzneiform, die für die perorale Applikation geeignet ist, vorliegen.

4. Verwendung nach einem der Ansprüche 2 oder 3, dadurch gekennzeichnet, daß der Gehalt an Extrakt aus Rhizoma zingiberis zwischen 50 und 200 mg pro Einnahmeeinheit liegt.

5. Verwendung nach Anspruch 4, dadurch gekennzeichnet, daß der Gehalt an Extrakt aus Rhizoma zingiberis bei 100 mg pro Einnahmeeinheit liegt.

6. Verwendung nach einem der Ansprüche 2 ibs 5, dadurch gekennzeichnet, daß der Gehalt an Extrakt aus Ginkgo bilobae zwischen 10 und 100 mg pro Einnahmeeinheit liegt.

7. Verwendung nach Anspruch 6, dadurch gekennzeichnet, daß der Gehalt an Extrakt aus Ginkgo bilobae bei 40 mg pro Einnahmeeinheit liegt.

8. Verwendung nach einem der Ansprüche 2 bis 7, dadurch gekennzeichnet, daß der Extrakt aus Rhizoma zingiberis durch eine Ethanol/Wasser- oder Aceton/Wasser-Extraktion gewonnen ist und im wesentlichen als Oleoresin vorliegt.

9. Verwendung nach einem der Ansprüche 2 bis 7, dadurch gekennzeichnet, daß der Extrakt aus Rhizoma zingiberis durch Extraktion mit CO₂ gewonnen ist.

10. Verwendung nach Anspruch 8 oder 9, dadurch gekennzeichnet, daß das Oleoresin oder der CO₂-Extrakt aus Rhizoma zingiberis durch Mikroverkapselung, Granulierung oder Lyophilisierung in eine geeignete galenische Form überführt ist.

## Claims

1. Use of a composition containing active substances from Rhizoma zingiberis and Ginkgo bilobae for preparing a drug for treatment of anxiety states.

2. Use according to claim 1, characterised in that the composition contains a combination of extracts of Rhizoma zingiberis and Ginkgo bilobae.

3. Use according to claim 2, characterised in that the active substances or extracts in an ingestion unit are present in capsule form or another solid or liquid medicinal form suitable for oral application.

4. Use according to claim 2 or 3, characterised in that the content of Rhizoma zingiberis extract is between 50 and 200 mg per ingestion unit.

5. Use according to claim 4, characterised in that the content of Rhizoma zingiberis extract is about 100 mg per ingestion unit.

6. Use according to any of claims 2 to 5, characterised in that the content of Ginkgo bilobae extract is between 10 and 100 mg per ingestion unit.

7. Use according to claim 6, characterised in that the content of Ginkgo bilobae extract is about 40 mg per ingestion unit.

8. Use according to any of claims 2 to 7, characterised in that the Rhizoma zingiberis extract is obtained by ethanol/water or acetone/water extraction and is substantially in oleoresin form.

9. Use according to any of claims 2 to 7, characterised in that the Rhizoma zingiberis extract is obtained by extraction with CO₂.

10. Use according to claim 8 or 9, characterised in that the oleoresin or CO₂ extract of Rhizoma zingiberis is converted into a suitable galenic form by micro-encapsulation, granulation or freeze-drying.

## Revendications

1. Utilisaion d'une composition contenant des substances actives de Rhizoma zingiberis et de Ginkgo bilobae pour la préparation d'un médicament pour le traitement des états d'anxiété.

2. Utilisation selon la revendication 1, caractérisée par le fait que la composition contient une combinaison d'extraits de Rhizoma zingiberis et de Ginkgo bilobae.

3. Utilisation selon la revendication 2, caractérisée par le fait que les substances actives ou les extraits se présentent en dose unitaire sous forme de gélules ou sous une autre forme galénique solide ou liquide, convenant pour l'application par voie orale.

4. Utilisation selon l'une des revendications 2 ou 3, caractérisée par le fait que la teneur en extrait de Rhizoma zingiberis se situe entre 50 et 200 mg par dose unitaire.

5. Utilisation selon la revendication 4, caractérisée par le fait que la teneur en extrait de Rhizoma zingiberis est de 100 mg par dose unitaire.

6. Utilisation selon l'une des revendications 2 à 5, caractérisée par le fait que la teneur en extrait de Ginkgo bilobae se situe entre 10 et 100 mg par dose unitaire.

7. Utilisation selon la revendication 6, caractérisée par le fait que la teneur en extrait de Ginkgo bilobae est de 40 mg par dose unitaire.

8. Utilisation selon l'une des revendications 2 à 7, caractérisée par le fait que l'extrait de Rhizoma zingiberis a été obtenu par une extraction à l'éthanol/eau ou à l'acétone/eau et se présente essentiellement sous forme d'oléorésine.

9. Utilisation selon l'une des revendications 2 à 7, caractérisée par le fait que l'extrait de Rhizoma zingiberis est obtenu par extraction avec du CO₂.

10. Utilisation selon la revendication 8 ou 9, caractérisée par le fait que l'oléorésine ou l'extrait au CO₂ de Rhizoma zingiberis est converti en une forme galénique appropriée par microencapsulation, granulation ou lyophilisation.
